# EUROPEAN PATENT APPLICATION

(11) **EP 4 170 742 A1**
(43) Date of publication of application: **26.04.2023**
(21) Application number: 21827965.1
(22) Date of filing: 22.06.2021
(51) Int. Cl.: H01M 4/04, A61N 1/04, C12M 1/42, B82Y 40/00, G01N 27/02, G01N 33/483

(54) **NANOSTRUCTURED ELECTRODES FOR THE ELECTRICAL STIMULATION OF CULTURED CELLS, AND DEVICES, SYSTEMS AND PROCEDURES ASSOCIATED THEREWITH**

(30) Priority: 23.06.2020 ES 202030626
(71) Applicant: Consejo Superior de Investigaciones Científicas (CSIC), 28006 Madrid (ES); Universidad Autónoma de Madrid, 28049 Madrid (ES); University of Florida Research Foundation, Inc. (UFRF), Gainesville, FL 32611-5550 (US)
(72) Inventor: MOBINI, Sahba, 28760 Madrid (ES); GARCÍA MARTÍN, José Miguel, 28760 Madrid (ES); GONZÁLEZ SAGARDOY, María Ujué, 28760 Madrid (ES); MARTÍN GONZÁLEZ, Maria Soledad, 28760 Madrid (ES); CABALLERO CALERO, Olga, 28760 Madrid (ES); GARCÍA MARTÍNEZ, Jorge M., 28760 Madrid (ES); DÍEZ GUERRA, Francisco Javier, 28760 Madrid (ES); PATRICK, Erin E, Gainesville, Florida 32605 (US)
(74) Representative: Tribalyte Ideas
(86) International application number: PCT/ES2021/070462
(87) International publication number: WO 2021/260248

(57) **Abstract**

The present invention relates to an electrical cell stimulation electrode (1), adapted for the insertion thereof into a miniaturised cell culture mini-well (3) plate (2), comprising at least one material disposed as a substrate (4) and an electrically conductive material disposed on said substrate (4). Advantageously, the electrically conductive material is formed by metallic nanopillars (5), deposited on the substrate (4) by means of glancing angle deposition (GLAD) with magnetron cathode sputtering. The invention likewise relates to different devices, systems, uses and procedures associated with said electrodes (1).

## Description

### FIELD OF THE INVENTION

The present invention belongs to the field of electrical stimulation techniques for cultured cell. More specifically, the invention relates to a new technology relating to nanostructured electrodes for carrying out said stimulation, as well as to various devices, systems and methods comprising the mentioned electrodes.

### BACKGROUND OF THE INVENTION

Electrical stimulation (ES) techniques are used today for several purposes, among which are the healing of damaged tissues, such as bones, skin, muscles, and nerves, in *in vivo* clinical and pre-clinical applications. Furthermore, it is known that electrical stimulation can induce *in vitro* regenerative responses in cultured cells, such as an increase in the expression of pro-regenerative growth factors, and of genetic markers and proteins.

Electrical stimulation is an appealing technique for basic, clinical and translational research, as it provides a controllable and scalable physical tool to improve regenerative and healing responses in a number of tissues and cell types that cannot heal naturally. The potential result of this field of research points to the development of implantable therapeutic electrical stimulation devices, configured to administer an optimised dose of electrical signals, which provides an improved and accelerated healing of damaged tissues. This possibility is of great interest, for example, in the case of traumatic or degenerative diseases. Future applications of this technology likewise comprise the study of uses of electrical stimulation to maintain the muscular and nervous system in reduced gravity, or to provide improved cognitive capabilities, among others.

However, in order to achieve the possible potential of electrical stimulation-based therapies the development of specific techniques, adapted to each type of tissue, is still needed. This is primarily due to the fact that each tissue has unique biological complexities and a characteristic biophysics, and so do their underlying molecular mechanisms. In this area, various electrical stimulation devices, configured to influence the cell functions associated with the proliferation, differentiation, migration, and secretion of growth factors, have been developed in recent years. Said devices (see, for example, Leppik L., Zhihua H., Mobini S., et al., "Combining electrical stimulation and tissue engineering to treat large bone defects in a rat model", Sci. Rep. 2018, 8(1) and the references mentioned therein) comprise the use of pairs of platinum electrodes, with which an electric field is applied to a multiwell cell culture plate. Although this type of devices is now widely used, its performance still has technical shortcomings that have not been resolved, which can be summarised in three main limitations described below.

First, one of the key challenges of electrical stimulation devices is the precise definition of the electrical parameters of their electrodes. Said parameters are related to the amplitude and shape of the applied voltage signal and to the geometry of the cell culture chambers or housings, as well as to the electrical properties of the materials that make up the electrodes. Therefore, in order to obtain a proper definition of the electrical stimulation parameters and thus a precise control of the electric field experienced by the cultured cells, it is necessary that the electrodes can be manufactured in a reproducible way, providing stable and optimal electrical properties. In this way, the actual parameters (local electric field value, voltage, current) of the actual electrical stimulation applied at the cellular level can be reliably predicted for each experiment. Also, electrodes must be sterilizable and durable, as their surface and electrical properties generally affect their impedance and, consequently, this can significantly affect the electrical signal delivered to the cells. For example, the most common monolithic electrodes, usually made of metallic or carbon materials, exhibit surfaces with a variable and non-reproducible structure and are altered over time, as are their electrical properties. Therefore, replacing these electrodes with new ones, or changing from one type to another, alters in an uncontrollable way the results of the experiments carried out. Carbon electrodes also have a high protein absorption, which considerably influences their performance, even after a single experiment, making it almost impossible to reproduce the same conditions and results each time.

Secondly, another limitation of electrical stimulation devices stems from the cell culture systems into which they are integrated, typically comprising standard-sized cell culture plates with multiple wells. While this design has its own advantages and reasonably satisfies the conditions of some experiments that require more volume, it is not suitable for experiments that need to be performed in smaller volumes, due to limitations in the use of expensive and/or scarce reagents (e.g. growth factors and specific culture media used in neuroscience experiments), or in the number of initial cells available.

Thirdly, known electrical stimulation devices are mainly based on the use of multiwell culture plates made of plastic, e.g. polystyrene, which are not suitable for the observation of cultured cells by high-quality microscopy techniques, in contrast to traditional glass coverslips, which do allow high-quality live imaging.

The present invention is intended to solve the aforementioned problems by means of a novel technology relating to nanostructured electrodes, suitable for preferred use in cell culture configurations consisting of mini-wells (the width, height, or depth dimensions of which are typically in the order of a tenth of mm) on microscopy slides (with a standard size, 75 mm long and 25 mm wide). Said electrodes and the mini-cell culture chambers are advantageously integrated in a "miniaturised electro-bioreactor". The set of metallic nanostructured electrodes, which are well-characterised as a result of their manufacture under precise conditions, is capable of providing an electrical stimulation with adjustable and reproducible parameters.

### BRIEF DESCRIPTION OF THE INVENTION

In order to solve the aforementioned limitations and problems, a first object of the invention relates to an electrode, adapted for the insertion thereof into a miniaturised cell culture plate (size corresponding to a microscopy slide) and, more specifically, for the insertion thereof into one or more cell culture mini-wells of said plate. To that end, the electrode of the invention is preferably manufactured from a material which is disposed as a substrate, on which an electrically conductive material is deposited, forming a complex nanostructure acting as an effective electrical exchange surface of said electrode.

Advantageously in the invention, the electrically conductive material of the electrode is formed by metallic nanopillars (also referred to as nanocolumns), which are deposited on the substrate through a glancing angle deposition (GLAD) technique. Said deposition technique consists of the relative inclination of the substrate with respect to a source of evaporation of metal particles or atoms, which generates a nanostructured surface on the substrate in the form of columns or pillars throughout the deposition process. This inclination of the substrate with respect to the source is called the GLAD angle (also known as the α angle in the literature). It has to be equal to or greater than substantially 70° for columns to form due to the shadowing effect.

It is known that GLAD techniques can be carried out with different known physical vapour deposition processes, such as for example thermal evaporation, electron beam bombardment or magnetron cathode sputtering. However, the present invention relates only to the use of the latter technique, magnetron cathode sputtering, for the manufacture of the electrodes, given its higher efficiency in production costs as well as its lower environmental impact compared to other physical vapour deposition techniques.

In turn, the angle formed by the columns/pillars with respect to the normal to the substrate (usually referred to in the literature as β) will always be less than the GLAD or α angle, the two being traditionally related by the "tangent rule": tan(β)=0.5*tan(α). However, it is now known that this rule overestimates the value of β, and that its prediction is complex, since it depends on many factors, such as the materials used, the degree of ballistic regime in which it is used, whether it is in strong or weak plasma conditions, etc. These factors are generally regulated by the gas pressure and the working power, as well as by experimental validation and/or modelling. Furthermore, in cases where the substrate is rotating around an axis perpendicular to its surface, the structures produced will not be inclined, but will always grow vertically, i.e. perpendicular to the substrate (β =0° for any α). Obviously, the rotation of the substrate is economically more costly, as it requires an additional motor in the equipment.

In the scope of the invention, the preferred working conditions comprise, therefore, the use of the following parameters: glancing angle of the substrate α ≥70° and gas pressure during deposition P ≤ 5 Pa, which allows obtaining an inclination β of the nanocolumns of 0° to 70°measured with respect to the normal to the substrate.

In this way, GLAD can provide both straight nanopillars on the substrate and more complex structures, in case the substrate moves during the deposition of the metal particles, which can lead to zig-zag, V-shaped, etc. structures. GLAD techniques are generally known in the state of the art of material deposition techniques. However, the invention described herein claims the use of such techniques for the manufacture of electrodes for the electrical stimulation of cultured cells, as well as the electrodes obtained by such manufacture and their manufacturing processes.

Obtaining cell electrical stimulation electrodes by means of GLAD with magnetron cathode sputtering is suitable for the large-scale manufacture of said electrodes, in an industrially scalable and environmentally friendly manner. Furthermore, as will be seen hereinbelow, the electrodes obtained by means of this technique exhibit very stable electrical properties, both in the electric fields generated and in the reproducibility of their properties when they are mass produced.

As a result of the proposed nanostructures, the electrodes of the invention provide a considerably larger effective surface area than the electrodes used to date, which reduces the impedance of the electrical stimulation system and increases the capacitive part of this impedance. This results in two advantageous features of great importance for electrical stimulation both *in vivo* and *in vitro*: on the one hand, the amplitude of the voltage signal required to obtain the desired voltage or electric field value inside the mini-culture chamber is lower, and on the other hand, the electrical charge injected into the system is higher without the need to raise the voltage above the breakdown value of water molecules, which are essential molecules in biological systems.

In a preferred embodiment of the invention, the metal particles forming the nanopillars of the electrodes comprise biocompatible metals, such as titanium, gold, or platinum, separately or in combination. In turn, the substrate of the electrodes preferably comprises silicon, quartz, glass, or glass/quartz coated with a thin film of a transparent conductive oxide (TCO), such as fluorine doped tin oxide (FTO) or indium tin oxide (ITO).

In relation to the characteristics of the nanopillars, these are preferably disposed on the substrate with a relative glancing angle (β) of, at most, 70°. Said glancing angle β of the nanocolumns with respect to the normal to the substrate, comprised between 0° and 70°, is considered advantageous with respect to other inclinations, since it favours the electric properties of the electrodes.

Moreover, in relation to the shape and size properties of the nanopillars, the lengths thereof primarily depend on the deposition times of the metal particles or atoms. Typical ranges of lengths that can be achieved by means of GLAD are comprised between 10 and 4000 nm. Preferably, the length considered the most suitable for the electrical stimulation electrodes applications described herein is comprised between 10 and 300 nm. Likewise, the diameters and the separation of the nanopillars depend on several factors, such as the properties of the substrate (for example, its roughness), the mass of the deposited atoms , the deposition energy (typically tens of eV) or the atomic diffusion. Said factors can be controlled within certain limits during the nanopillar formation process, causing the mentioned diameters and their separation, for the applications of the invention, to be comprised between 20 and 120 nm for gold, between 30 and 160 nm for Ti, and between 10 and 100 nm for Pt. Preferably, the range generally considered most suitable for the nanopillars of the invention is comprised between 30 and 100 nm.

A second object of the present invention relates to a device for the electrical stimulation of cultured cells, comprising a disposition of the electrodes according to any of the embodiments described herein, wherein said electrodes are adapted for the insertion into plates or cell culture wells (either of standard size or miniaturised ones, whose size corresponds to a microscopy slide).

In a preferred embodiment of the device of the invention, the electrodes are disposed in pairs, and adapted for the insertion of each of said pairs into a mini-well (the side of which would be 10 mm x 10 mm) of a miniaturised cell culture plate the side of which lateral corresponds to a microscopy slide (75 mm x 25 mm). In this configuration, the electric field and current density inside the cell culture mini-well have a high spatial stability, which allows these parameters to be easily calculated locally (relevant to know the real parameters experienced by the cells) by means of computational modelling and experimental verification.

In another preferred embodiment of the device of the invention, the electrodes are attached to a printed circuit board (PCB) comprising a connection to a function generator and/or to an electrical power source.

In another preferred embodiment of the device of the invention, the electrodes are adhered to the PCB by means of a conductive epoxy adhesive.

In another preferred embodiment of the device of the invention, said device further comprises a transparent upper cover, which holds the PCB and the electrodes.

In another preferred embodiment of the device of the invention, the PCB comprises independent connections between at least two electrodes and the function generator/electrical power source.

In another preferred embodiment of the device of the invention, the PCB comprises a set of holes aligned with the mini-wells of the cell culture plate.

A third object of the present invention relates to a system for the electrical cell stimulation *in vitro*, comprising a device according to any of the embodiments described herein, in combination with a disposition of one or more miniaturised cell culture plates (generally of the size corresponding to a light microscopy slide or the like).

In a preferred embodiment of the system of the invention, the cell culture mini-wells are integrated in a monolithic piece, and divided by walls or partitions formed in said piece.

In another preferred embodiment of the system of the invention, the cell culture micro-plates exhibit a prism or cube shape, and the electrodes are disposed in pairs, wherein each of the electrodes forming said pairs is disposed on opposite sides of a plate or of a corresponding cell culture mini-well.

In another preferred embodiment of the system of the invention, the miniaturised plates and/or the cell culture mini-wells comprise a transparent lower surface, suitable for use with a microscope for monitoring a culture housed in any of the plates/cell culture wells. More preferably, said transparent lower surface is detachable with respect to the disposition of the plates/cell culture wells. Said lower surface can consist of a standard light microscopy slide or, in order to achieve the maximum image quality offered by immersion objectives, the glass surface should typically be 0.17 mm thick (coverslip no. 1.5).

As a result of these characteristics, the described electrical stimulation system provides a suitable tool for obtaining high-quality images of cultured cells, either post-processed or in real time, while said cells are subjected to electrical stimulation.

As mentioned, the need for such devices and systems on the market is currently very high. In this context, the present invention proposes a technology that not only improves the control of the applied electrical stimulation parameters but is also very easy to use (in a plug-and-play approach), allowing the user to simultaneously apply well-characterised and fully reproducible electrical conditions in multiple cell culture mini-wells. Since the system of the invention is adapted for use in combination with the aforementioned miniaturised cell culture plates (lateral size corresponding to a microscope slide, 75 mm x 25 mm), it saves significant space as it can be stacked in an incubator. In addition, it reduces the consumption of biological materials, due to its miniaturised design (achieved by the small size of its electrodes and mini-wells), but is at the same time large enough to maintain cultures under optimal conditions for several days.

A fourth object of the invention relates to the use of GLAD with magnetron cathode sputtering for the manufacture of cell stimulation electrodes according to any of the embodiments described herein.

A fifth object of the invention relates to a method of manufacturing cell stimulation electrodes, comprising disposing a first material as a substrate and depositing a second electrically conductive material on said substrate, by means of GLAD with magnetron cathode sputtering, forming a disposition of nanopillars thereon.

### DESCRIPTION OF THE DRAWINGS

Figure 1 shows the electrical cell stimulation system of the invention, according to a preferred embodiment of said system.
Figure 2 shows images taken by electron microscopy illustrating the nanostructure of the electrodes of the invention, according to a preferred embodiment thereof.

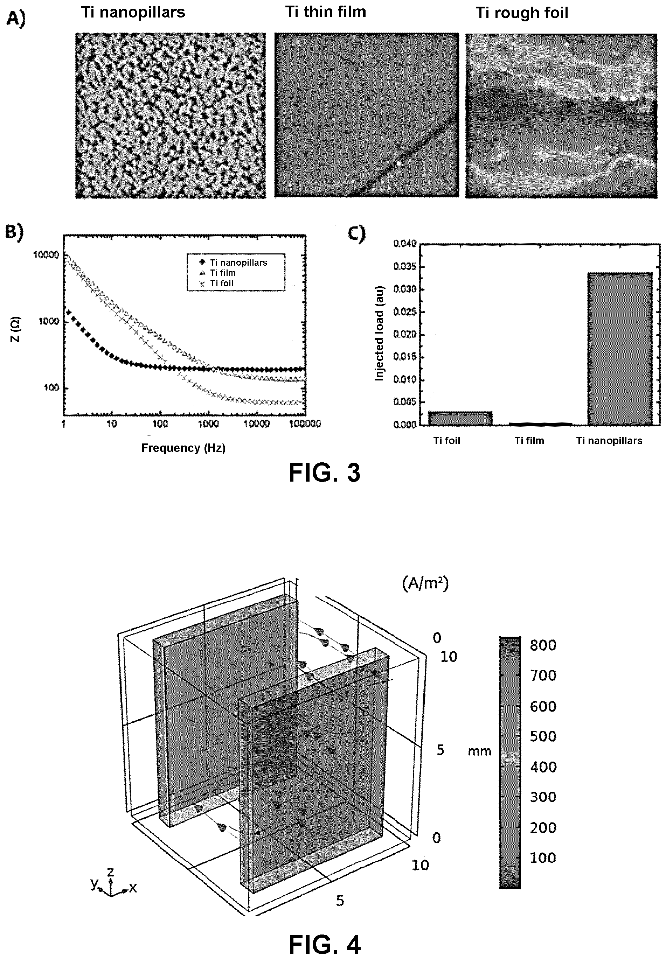

Figure 3 shows a comparison of operating conditions for three types of titanium metal electrodes, wherein one of them is formed by the claimed nanopillar structure (Figure 3a, left image) and the others correspond to titanium foils of different thicknesses (without nanostructure), wherein one of these foils has a low roughness (Figure 3a, central image) and the other one has a high roughness (Figure 3a, right image). Figure 3b depicts the impedance of the three types of electrodes based on the frequency of the electrical signal applied to them. Figure 3c depicts the charge injection capacity of the three types of electrodes used.
Figure 4 shows a computational model of the distribution of the current density within the cell culture area, for a configuration of a pair of electrodes according to the invention, applied to a cube-shaped cell culture well.
Figure 5 shows the results of a numerical model of the transient behaviour of a Ti nanopillar electrode system according to the invention, applied to an electrolyte (Figure 5a). These results are compared with similar results obtained for electrodes made of Ti thin films (Figure 5b).
Figure 6 shows the results of a numerical model of the transient behaviour of an Au nanopillar electrode system according to the invention, applied to an electrolyte (Figure 6a). These results are compared with similar results obtained for electrodes made of Au thin films (Figure 6b).

### Reference numbers of the drawings:

(1) Electrodes.
(2) Cell culture plate.
(3) Cell culture wells.
(4) Substrate.
(5) Nanopillars or nanocolumns.
(6) Upper cover.
(7) PCB.
(8) Connection electric.
(9) Holes.
(10) Dividing walls.
(11) Glass lower plate.

### DETAILED DESCRIPTION OF THE INVENTION

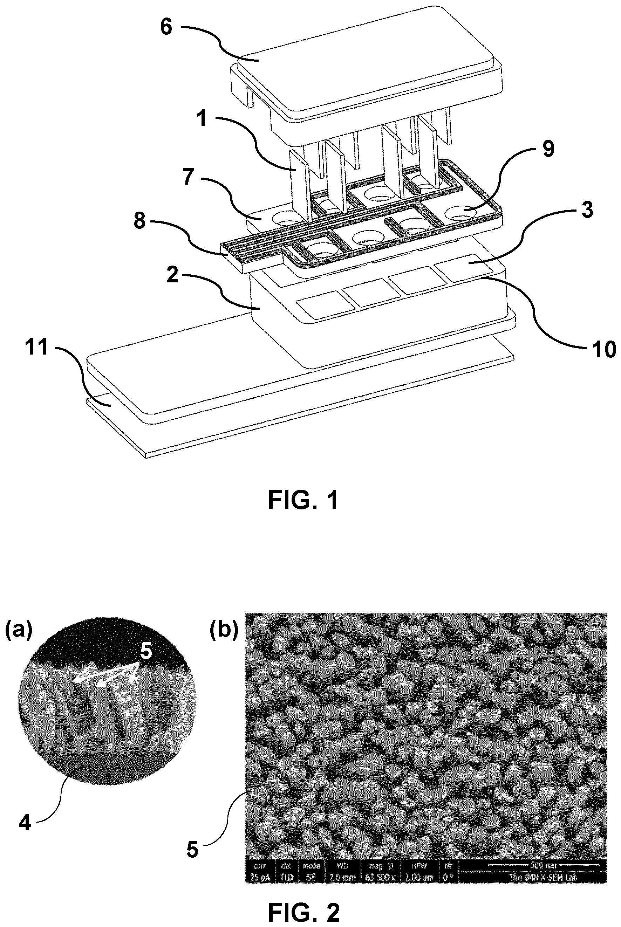

As described in the preceding sections, as well as in the examples shown in Figures 1-6 herein, a first object of the invention relates to an electrode (1) adapted for the insertion thereof into a miniaturised cell culture plate (2) and, more specifically, for the insertion thereof into one or more cell culture mini-wells (3) of said plate (2) (see Figure 1). To that end, the electrode (1) of the invention is preferably manufactured from a material disposed as a substrate (4), on which an electrically conductive material, preferably formed by metallic nanopillars (5), is deposited (Figure 2), and adopting a nanostructure acting as an effective electrical exchange surface of said electrode (1). Advantageously, the metallic nanopillars (5) of the electrode (1) are deposited on the substrate (4) by means of GLAD with magnetron cathode sputtering, which favours the reproducibility of the properties of the electrodes for their large-scale manufacture.

Obtaining electrical cell stimulation electrodes by means of GLAD is a unique feature that provides a lower impedance but higher capacitive component compared to other electrodes, which is a great advantage for both *in vitro* and *in vivo* electrical stimulation systems. Advantageously, this feature allows the amplitude of the voltage signal needed to obtain the desired voltage or electric field value inside the mini-culture chamber to be lower as well as delivering a higher charge to the system without breaking down water molecules, which are essential molecules in biological systems.

In a preferred embodiment of the invention, the metal particles forming the nanopillars (5) of the electrodes (1) comprise biocompatible metals, such as titanium, gold, or platinum, separately or in combination. In turn, the substrate (4) of the electrodes (1) preferably comprises silicon, quartz, glass, glass or quartz coated with a thin film of a transparent conductive oxide (TCO), such as fluorine doped tin oxide (FTO) or indium tin oxide (ITO).

Figure 1 of this document shows an exploded view of the system for electrical stimulation of cell cultures of the invention. In addition to the electrodes (1), the plate (2) and the wells (3), the figure shows other main elements of the system. Said elements include, for example, an upper cover (6), preferably made of transparent polyester, which allows light to pass through to obtain real-time images when the stimulation is being performed. Said upper cover (6) is preferably adapted to also house a PCB (7) to which the electrodes (1) are electrically connected. In this way, the assembly formed by the lid (6), the PCB (7) and the electrodes (1) forms an electrical stimulation device interchangeable with a conventional flat cover, when stimulation of the cultures is not required, and at the same time avoiding contamination of the cultures.

Preferably, the PCB (7) comprises a connection (8) for connecting the stimulation device to a function generator or to a power source. Likewise, the circuit of the PCB (7) is preferably configured to connect the electrodes (1) to said generator/source independently. In this way, the plate (2) wells (3) can thereby be stimulated with the same or different signals, as required. Likewise, the PCB (7) is configured to effectively hold the electrodes (1), for example through adhesive means such as conductive epoxy resins. In other embodiments, it is also possible to perforate holes (9) in suitable regions of the PCB (7) to allow the passage of light while taking images of the cell cultures housed in the wells (3) and which, as described, are preferably separated by dividing walls (10). Additionally, the lower part (11), corresponding to the bottom of the cell culture plate (2), which is where the cells are cultured, will be made of an optical-grade transparent material (for example glass) suitable to allow an optical image to be taken. Furthermore, said lower plate (11) of the system of the invention can be permanently adhered to the culture wells (3) or can be removable. When the lower part (11) is removable, it may consist of a standard light microscopy slide or a coverslip (0.17 mm thick glass) if high-quality microscopy is required. However, in different embodiments of the invention, the lower part (11) of the plate (2) can have a larger surface than that occupied by the set of wells of part (2). In any case, a prolongation of the base of part (2) to completely match up the surfaces of the lower part (11) and of the rest of the plate (2) will result in a generally more robust configuration, accordingly reducing the risk of the slide/coverslip breaking.

The electrodes (1) of the invention can be characterised in terms of their electrical properties in operating conditions (i.e., in an operating situation when applied to the cultures housed in the wells (3) of the system). Thus, for example, Figure 3 of the present document shows a comparison of said properties for three types of titanium metal electrodes, wherein one of them is formed by the claimed nanopillar structure (5) (Figure 3a, left image), and the others correspond to titanium foils of different thicknesses (without nanostructure), wherein one of said foils has a low roughness (Figure 3a, central image) and the other one has a high roughness (Figure 3a, right image). As can be seen in Figure 3b, the electrodes (1) of the invention show a significant reduction in the impedance of the system, particularly for low electrical signal frequencies. Likewise, the electrodes (1) also have a significant increase in the charge injection capacity (Figure 3c), as a consequence of the increase in its effective area compared with the other two types of laminar structures.

The surface roughness of the three Ti electrodes, measured by atomic force microscopy, for the nanopillars (5) has a value of Rq = 26 nm, while for the thin film it has a value of Rq = 1.2 nm, and for the thicker foil it has a value of Rq = 66 nm. The electrical impedance of Ti has been measured in artificial cerebrospinal fluid using electrochemical impedance spectroscopy and the charge injection capacity has been measured by cyclic voltammetry at a sweep speed of 100 mV/s, and a voltage range of 2V. Tests of the above properties on other metals, such as Au and Pt, confirm results similar to those obtained for Ti.

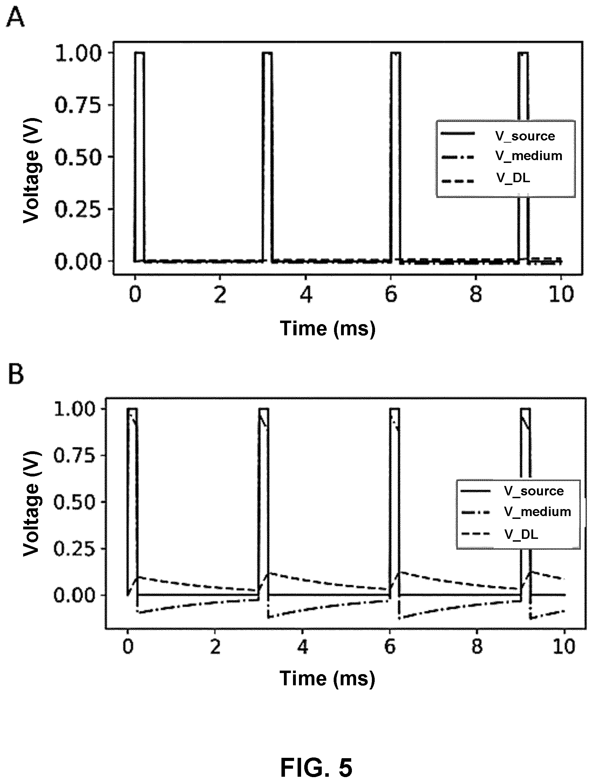

To illustrate the electrical stability properties of the electrodes (1) of the invention, Figure 4 shows a computational simulation performed by means of COMSOL Multiphysics^{®} modelling software, applied to a pair of electrodes in the form of square and flat plates, disposed in a cube-shaped culture well (3). Said figure shows that the configuration of the electrodes (1) depicted provides a homogeneous and stable electric field and current density (the simulation has been performed for an electrode current of 0.001 A, applied to Ti electrodes and with a phosphate buffer saline electrolyte, or PBS). This property is significant since, in general, conventional electrode designs based on the use of rods and wires are not able to provide a uniform field in the culture volume. Therefore, the stability of the electrical properties of the electrodes of the invention allows electrical stimulation protocols to be designed with much greater precision than with known electrodes. In this , when applying electrical stimulation through voltage-controlled methods, it is of particular importance to be able to understand how the metal/electrolyte interface filters the applied signal, in order to predict the amount and temporal nature of the electric field actually experienced by the medium. This is because the resulting voltage experienced by the medium (V_{medium}) may not be the same as the waveform of the applied voltage (V_{source}), after being filtered by the capacitance of the electrical double layer formed at the metal/electrolyte interface (V_{DL}). For the case of pulsed applied voltages, the transient nature of the voltage across the medium depends on the time constant, τ, of the system, which results from the combination of its resistive and capacitive elements, and depends on the geometry of the electrodes (1) and the wells (3). Figure 5 shows the response obtained for electrodes (1) of Ti nanopillars (5) (Figure 5a) and for thin film systems (Figure 5b) when applying a voltage with a square waveform of 1 V amplitude in pulse trains of 200 µs duration and 3 ms period. Said response has been calculated by considering the electrodes as equivalent circuits consisting of a series resistor with a constant phase element representing the capacitance of the electrical double layer at the metal/electrolyte interface. The values assigned to the equivalent circuit elements have been obtained from experimental electrochemical impedance spectroscopy measurements performed on both types of electrodes. As a consequence of the fact that the double layer capacitance of the Ti nanopillars (5) system is an order of magnitude higher than that of the Ti thin film system, the electrodes (1) based on nanopillars (5) filter the source signal (V_{source}) less than in the thin film system, producing a voltage drop in the medium (V_{medium}) which is much closer to the source signal. These results therefore show that with textured electrodes (1) with nanopillars (5), it is easier to provide alternating electric fields or pulse sequences with well-defined shape and amplitude in the culture medium at the cellular level, since the double layer effects of the metal/electrolyte interface are minimised.

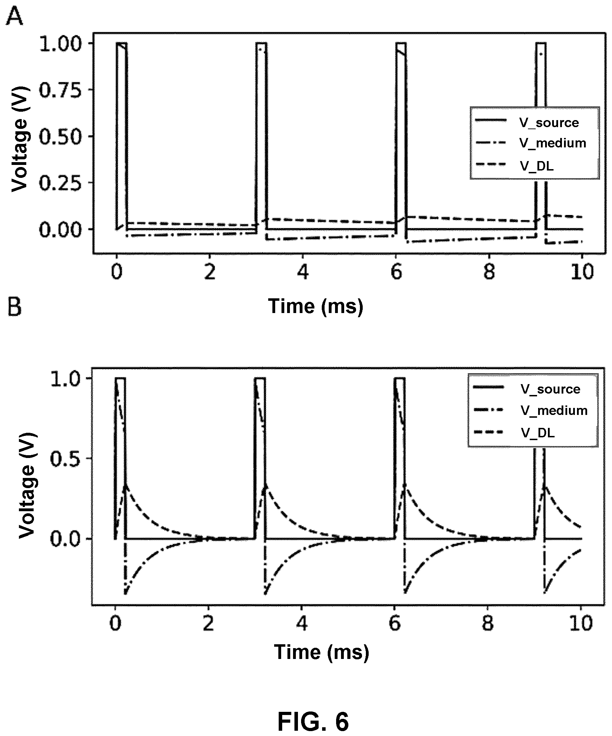

Figure 6 shows results similar to those depicted in Figure 5, generated for electrodes (1) with Au nanopillars (5) (Figure 6a) and for Au thin film systems (Figure 6b).

## Claims

1. A device for the electrical cell stimulation, comprising:
- a cell culture well (3) plate (2); and
- an electrode (1) adapted for the electrical stimulation of a cell culture housed in one or more wells (3) of the plate (2), wherein the electrode (1) comprises at least one material disposed as a substrate (4) and an electrically conductive material disposed on said substrate (4);
**characterised in that** the electrically conductive material is formed by metallic nanopillars (5), deposited on the substrate (4) by means of GLAD with magnetron cathode sputtering.

2. The device according to the preceding claim, wherein the metallic nanopillars (5) comprise titanium, gold, and/or platinum.

3. The device according to any of the preceding claims, wherein the substrate (4) comprises silicon, quartz, glass, glass or quartz coated with a thin film of a transparent conductive oxide (TCO), such as fluorine doped tin oxide (FTO) or indium tin oxide (ITO).

4. The device according to any of the preceding claims, wherein the disposition of the nanopillars (5) on the substrate (4) has a glancing angle comprised between 0°-70° with respect to the normal of said substrate (4), which corresponds to a glancing angle deposition α ≥ 70° and a gas pressure during said deposition P <_ 5 Pa.

5. The device according to any of the preceding claims, wherein the length of the nanopillars (5) is comprised between 10 and 4000 nm.

6. The device according to the preceding claim, wherein the length of the nanopillars (5) is comprised between 10 and 300 nm.

7. The device according to any of the preceding claims, wherein the diameter and/or the mean separation of the nanopillars (5) are comprised between 30 and 100 nm.

8. The device according to any of the preceding claims, comprising a plurality of electrodes (1) adapted for the insertion thereof into a corresponding disposition of cell culture well (3) plates (2).

9. The device according to the preceding claim, wherein the electrodes (1) are disposed in pairs and adapted for the insertion of each pair of electrodes (1) into a plate (2) or a corresponding cell culture well (3).

10. The device according to any of claims 8-9, wherein the electrodes (1) are fixed to a PCB (7) comprising a connection (8) to a function generator and/or to an electrical power source.

11. The device according to the preceding claim, wherein the electrodes (1) are fixed to the PCB (7) by means of a conductive epoxy adhesive.

12. The device according to any of claims 10-11, further comprising a transparent upper cover (6), which houses the PCB (7) and the electrodes (1).

13. The device according to any of claims 10-12, wherein the PCB (7) comprises independent connections (8) between at least two electrodes (1) and the function generator/electrical power source.

14. The device according to any of claims 10-13, wherein the PCB (7) comprises a set of holes (9) aligned with corresponding openings of the plate (2) or with the cell culture wells (3).

15. A system for electrical cell stimulation, comprising a device according to any of claims 8-14, in combination with a disposition of plates (2) or of cell culture mini-wells (3).

16. The system according to the preceding claim, wherein the plate (2) and the cell culture wells (3) are integrated in a monolithic piece and divided by walls (10) formed in said piece.

17. The system according to any of claims 15-16, wherein the plates (2) and/or the cell culture wells (3) exhibit a prism or cube shape, and the electrodes (1) are disposed in pairs, wherein each of the electrodes (1) of the pair is disposed on corresponding opposite sides of the plates (2) or of the cell culture wells (3).

18. The system according to any of claims 15-17, wherein the plates (2) and/or the cell culture wells (3) comprise a transparent lower surface (11), suitable for use with a light microscope, for monitoring a culture contained in the plates (2) or the cell culture wells (3).

19. The system according to the preceding claim, wherein the transparent lower surface (11) is detachable with respect to the plate (2) or the cell culture wells (3).

20. Use of GLAD with magnetron cathode sputtering for the manufacture of cell stimulation electrodes (1).

21. A method of manufacturing cell stimulation electrodes (1), comprising:
- disposing a first material as a substrate (4) and depositing a second electrically conductive material on said substrate (4), by means of GLAD with magnetron cathode sputtering, forming a disposition of nanopillars (5) thereon; and
- adapting said electrodes (1) for the insertion thereof into a cell culture well (3) plate (2).
